(19) Europäisches Patentamt
European Patent Office
Office européen des brevets

(11) Veröffentlichungsnummer: **0 100 974**
**B1**

(12) # EUROPÄISCHE PATENTSCHRIFT

(45) Veröffentlichungstag der Patentschrift: **23.04.86**

(51) Int. Cl.⁴: **A 23 K 1/18**

(21) Anmeldenummer: **83107481.0**

(22) Anmeldetag: **29.07.83**

(54) **Teilchenförmiges Futterzusatzmittel für Wiederkäuer.**

(30) Priorität: **14.08.82 DE 3230292**

(43) Veröffentlichungstag der Anmeldung: **22.02.84 Patentblatt 84/8**

(45) Bekanntmachung des Hinweises auf die Patenterteilung: **23.04.86 Patentblatt 86/17**

(84) Benannte Vertragsstaaten: **AT BE CH FR GB IT LI LU NL SE**

(56) Entgegenhaltungen:
**DE - A - 2 704 746**
**DE - A - 2 838 309**
**US - A - 3 941 818**
**US - A - 4 021 569**

(73) Patentinhaber: **Lohmann Tierernährung GmbH, Neufelder Strasse 24-28, D-2190 Cuxhaven (DE)**

(72) Erfinder: **Küther, Klaus H., Dr., Am Fuchsberg 23, D-2177 Wingst (DE)**
Erfinder: **Lübbe, Fritz, Dr., Schubertstrasse 30, D-2190 Cuxhaven (DE)**

(74) Vertreter: **UEXKÜLL & STOLBERG Patentanwälte, Beselerstrasse 4, D-2000 Hamburg 52 (DE)**

EP 0 100 974 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein teilchenförmiges Futterzusatzmittel für Wiederkäuer, das dazu dient, die Versorgung von Wiederkäuern mit essentiellen Aminosäuren zu verbessern.

In der Vergangenheit sind grosse Anstrengungen unternommen worden, die Versorgung von Wiederkäuern mit Nährstoffen wie beispielsweise Proteinen oder freien essentiellen Aminosäuren zu verbessern. Das Problem besteht dabei bei der oralen Verfütterung im wesentlichen darin, dass diese Stoffe, um für das Tier wirklich verfügbar zu sein, den Pansen praktisch unversehrt passieren müssen. Zu diesem Zweck werden beispielsweise Aminosäuren durch chemische Behandlung oder durch Umhüllung mit einer pansenfesten Beschichtung geschützt. Hierbei ergibt sich wiederum das Problem, dass zwar im Pansen ein Schutz der Aminosäure eintreten soll, dass dieser Schutz jedoch im anschliessenden Verdauungstrakt die Resorption der Aminosäuren nicht behindern darf. Das somit anzustrebende Ziel, im Pansen einen 100%igen Schutz und im anschliessenden Verdauungstrakt eine 100%ige Resorption zu erreichen, konnte bisher nicht annähernd erreicht werden. Es wurde vielmehr festgestellt, dass bei in bekannter Weise geschützten Aminosäuren der Schutz im Pansen unzureichend war oder die Resorption im anschliessenden Verdauungstrakt nicht den Erwartungen entsprach. Dies zeigte sich insbesondere bei in vivo-Versuchen, bei denen der Gehalt des Blutserums an essentiellen Aminosäuren in Abhängigkeit von der Zufütterung geschützter Aminosäuren untersucht worden ist. Es sei in diesem Zusammenhang auf einen Artikel von Dr. K. Küther in «Rinderwelt» Nr. 1, 1982, Seiten 3 bis 8 und auf Degussa-Informationsdienst für die Mischfutterindustrie 1/1979, S. 1-5 verwiesen, woraus sich der neueste Entwicklungsstand insbesondere auf die in der Fachwelt als für Wiederkäuer — vor allem für Hochleistungskühe — besonders bedeutend angesehene Aminosäure Methionin ergibt. Aus der Vielzahl der Vorschläge, die Methoden zum Schutz von biologisch aktiven Wirkstoffen im Pansen von Wiederkäuern betreffen, sei beispielsweise auf die DE-PS 22 12 568, die DE-PS 30 13 000, die US-PS 3 655 864, die GB-PS 1 137 214, die NO-PS 120 058 sowie auf die in diesen Druckschriften genannten Veröffentlichungen verwiesen.

Aufgrund der geschilderten Unzulänglichkeiten des Standes der Technik liegt der Erfindung die Aufgabe zugrunde, einen Futtermittelzusatz für Wiederkäuer zu liefern, der nicht nur in möglichst einfacher Weise herstellbar ist, sondern auch bei Verfütterung an Wiederkäuer eine bessere Kombination von Pansenpassage und Resorption im anschliessenden Verdauungstrakt von essentiellen Aminosäuren als bekannte Futterzusatzmittel liefert und dadurch die tatsächliche Verfügbarkeit der essentiellen Aminosäuren verbessert.

Zur Lösung dieser Aufgabe wird ein teilchenförmiges Futterzusatzmittel für Wiederkäuer der in den Patentansprüchen gekennzeichneten Art sowie ein Verfahren zu dessen Herstellung vorgeschlagen.

Wie bereits oben erwähnt, gilt nach dem bisherigen Erkenntnisstand Methionin als die für Wiederkäuer wichtigste essentielle Aminosäure, d.h. sie wird als erste limitierende Aminosäure angesehen. Dementsprechend soll die Erfindung im folgenden hauptsächlich anhand eines erfindungsgemässen Futterzusatzmittels beschrieben werden, das die Versorgung von Wiederkäuern mit Methionin verbessert.

Bekannt sind Zink-α-Aminosäure-Komplexsalze und insbesondere Zink-Methionin-Komplexsalze mit einem molaren Zink:Aminosäure-Verhältnis von 1:1 (siehe DE-AS 24 36 946, US-PS 3 463 858, US-PS 3 941 818 und US-PS 4 021 569). Hierbei handelt es sich um wasserlösliche Komplexsalze, deren einfach positiv geladenes Kation aus einem Zink-α--Aminosäure-Komplex besteht, während das Anion beliebig gewählt werden kann (siehe beispielsweise die in der DE-AS 24 36 946, der US-PS 3 941 818 und der US-PS 4 021 569 angegebenen Strukturformeln). In den angegebenen Druckschriften wird im wesentlichen darauf abgestellt, dass sich diese Zinkverbindungen als für den menschlichen und tierischen Körper besonders gut zugängliche Zinkquellen eignen. Mehr nebenbei ist ausserdem erwähnt, dass diese Verbindungen auch geeignete Substanzen für die Versorgung von Tieren mit essentiellen Aminosäuren sind. Auf die speziellen, beim Wiederkäuer auftretenden Probleme wird in diesen Druckschriften jedoch nicht eingegangen. Dies ist auch nicht verwunderlich, da es sich bei den beschriebenen Komplexsalzen um wasserlösliche Verbindungen handelt, die sich bei Verfütterung an Wiederkäuer im Pansen lösen und somit die sich anschliessenden Abschnitte des Verdauungstraktes nicht erreichen und dadurch auch nicht für eine zusätzliche Versorgung der Wiederkäuer mit essentiellen Aminosäuren sorgen können.

Weiterhin ist in der DE-A-27 04 746 die Verwendung des auch erfindungsgemäss geeigneten Zinkmethionats 1:2 als Bestandteil von Zusatzfuttermitteln beschrieben. Wie sich aus dem Oberbegriff des Patentanspruchs 1 und den Angaben in der Beschreibung ergibt, betrifft diese Druckschrift ein Mittel zur Wachstumsförderung und Futtereinsparung bei der Intensivmast von Kälbern (bei diesen ist das komplizierte Verdauungssystem der Wiederkäuer bekanntlich noch nicht voll ausgebildet, so dass die Pansenbeständigkeit von Futterkomponenten noch gar keine Rolle spielt), Rindern und Schafen. An keiner Stelle der DE-A-27 04 746 findet sich der geringste Hinweis darauf, dass wasserunlösliche Zinkkomplexe von essentiellen Aminosäuren, wie sie erfindungsgemäss verwendet werden, eine gewisse Pansenbeständigkeit aufweisen.

Erfindungsgemäss wurde nun überraschend gefunden, dass die sich bei Umsetzung von essentiellen Aminosäuren mit wasserlöslichen Zinksalzen bei geeignetem pH-Wert bildenden wasserunlöslichen Zinkkomplexe verhältnismässig pansenfest sind.

Mit sinkendem pH-Wert, spätestens nach salzsaurer Hydrolyse im Labmagen bei pH 2 - 4 lösen sich diese Verbindungen wieder in ihre Komponenten auf und stehen in den folgenden Abschnitten des Verdauungstraktes zur Resorption zur Verfügung. Dabei wird die Verfügbarkeit in den sich an den Pansen

anschliessenden Abschnitten des Verdauungstraktes und damit die anhand der Blutserumwerte verfolgbare Resorption noch erheblich dadurch gesteigert, dass man die Pansenfestigkeit erhöht, indem man die teilchenförmigen wasserunlöslichen Zink-Aminoäuren-Komplexe mit einer aus dem Stand der Technik bekannten, pansenfesten Matrix vermischt bzw. umhüllt.

Die Herstellung des erfindungsgemässen Futterzusatzmittels erfolgt in der Weise, dass man Methionin bei Raumtemperatur in Wasser durch Zugabe von Säure, z.B. Salzsäure in Lösung bringt. Anschliessend gibt man unter Rühren ein Zinksalz, z.B. Zinksulfat (wasserfrei oder hydratisiert) zu der Lösung, wobei sich das Salz rasch auflöst. Methionin und Zinksalz werden dabei in einem molaren Verhältnis von 2:1 eingesetzt. Die erhaltene, klare Lösung wird dann unter guter Durchmischung auf einen pH-Wert von 7,5 eingestellt, was vorzugsweise durch tropfenweise Zugabe einer Alkalilauge erfolgt. Nach Einstellung des pH-Wertes auf 7,5 wird noch eine Weile gerührt, bevor der ausgefallene Niederschlag abgetrennt, mit Wasser gewaschen und getrocknet wird. Die Herstellung von wasserunlöslichen Zinkkomplexen von Histidin, Phenylalanin und Valin erfolgt in analoger Weise. Ferner sei in diesem Zusammenhang erwähnt, dass die erhaltenen Niederschläge manchmal nicht nur aus den wasserunlöslichen Zinkkomplexen der Aminosäuren bestehen, sondern zum Teil erhebliche Mengen mitgefälltes Zinkhydroxid enthalten können.

Der in der oben beschriebenen Weise hergestellte, teilchenförmige, wasserunlösliche Zink-Methionin-Komplex wird mit einer pansenfesten Matrix vermischt bzw. umhüllt. Dies geschieht nach bekannten Verfahren, wie z.B. durch Sprühkristallisation (siehe DE-PS 24 51 510). Weitere geeignete Verfahren sind in den oben im Zusammenhang mit pansenfesten Beschichtungen angegebenen Druckschriften beschrieben. Insbesondere sei auf die DE-OS 30 13 000 und auf Chemie, Ingenieur und Technik 51 (1979), Nr. 11, Seiten 996 bis 998 und 45 (1973), Nr. 23, Seite 1339 hingewiesen. Selbstverständlich können auch Mischungen von Zinkkomplexen von zwei oder mehreren essentiellen Aminosäuren mit einer pansenfesten Matrix vermischt bzw. umhüllt werden.

Als pansenfeste Matrix zur Vermischung mit den erfindungsgemässen Zinkkomplexen oder zur Umhüllung der erfindungsgemässen Zinkkomplexe eignen sich die für diese Zwecke aus dem Stand der Technik bekannten Materialien. Zu diesen gehören beispielsweise Triglyceride, gesättigte Fettsäuren und deren Salze, Mischungen aus Mono-, Di- und Triglyceriden und Mischungen von Glycerintristearat mit flüssigen ungesättigten höheren Fettsäuren sowie Kombinationen der genannten Schutzmaterialien. Im einzelnen sei hierzu auf die eingangs erwähnten Druckschriften und die in diesen zitierten Veröffentlichungen verwiesen, wobei zusätzlich noch auf die DE-OS 28 38 278, die DE-OS 28 38 298 und die DE-OS 28 38 309 hingewiesen sei. Bezogen auf das Gesamtgewicht des erfindungsgemässen Futterzusatzmittels beträgt die Menge an pansenfester Matrix bis zu 90% und vorzugsweise 45 bis 65%. Im allgemeinen ist allerdings anzustreben, den Anteil der pansenfesten Matrix so gering wie möglich zu halten. Die Teilchengrösse des erfindungsgemässen Futterzusatzmittels aus Zinkkomplex und pansenfester Matrix liegt im Bereich von 0,3 bis 1,0 mm, wobei ein Bereich von etwa 0,3 bis 0,6 mm bevorzugt ist. Diese verhältnismässig geringe Teilchengrösse stellt gegenüber den aus der Praxis bekannten pansendurchtretenden Futterzusatzmitteln mit einer pansenfesten Matrix, welche mit einer Teilchengrösse um etwa 1,0 mm und mehr hergestellt werden, einen nicht unerheblichen Vorteil dar, da zwar einerseits die Oberfläche vergrössert, aber gleichzeitig die Passagezeit durch die Vormägen verkürzt wird. Hinzu kommen mischtechnische Vorteile, da diese feineren Partikel etwa die gleiche Grösse wie die Bestandteile von mehlförmigen (nicht pelletierten) Futtermischungen haben und somit nicht zum Entmischen bzw. Separieren neigen.

Ein erheblicher Nachteil der aus dem Stand der Technik bekannten pansendurchtretenden Futtermittelzusätze besteht ferner darin, dass diese nicht pelletiert werden können, da der chemische oder physikalische Schutz aufgrund der thermischen und mechanischen Einflüsse beim Pelletieren beeinträchtigt wird. Diese Eigenschaft begrenzt die Verwendung der bekannten pansendurchtretenden Futterzusatzmittel, da heutzutage Futtermittel (Mischfuttermittel) vorzugsweise in pelletierter Form hergestellt werden. Im Gegensatz dazu kann das erfindungsgemässe Futterzusatzmittel pelletiert werden, ohne dass dadurch eine erhebliche Beeinträchtigung der Schutzwirkung im Pansen eintritt. Dies beruht auf der grossen thermischen Stabilität der erfindungsgemässen Zinkkomplexe sowie auf deren Unempfindlichkeit gegen Wasser beim unmittelbar vor dem Pelletieren erfolgenden Konditionierungsprozess mit heissem Wasserdampf, so dass die geringfügige Beeinträchtigung der Schutzwirkung der Matrix durch das Pelletieren nicht ins Gewicht fällt.

Die Dosierung des erfindungsgemässen Futterzusatzmittels richtet sich selbstverständlich nach dem Bedarf des jeweiligen Tieres an den verschiedenen essentiellen Aminosäuren. So hat sich beispielsweise in Effektivitätsversuchen bei Kühen eine solche Menge an Futterzusatzmittel als geeignet erwiesen, die einer zusätzlichen Gabe von 1 bis 30 g Methionin/Tag und vorzugsweise etwa 15 g Methionin/Tag entspricht. Wegen des höheren Anteils des effektiv das Blutserum erreichenden Methionins ist zu erwarten, dass die Tagesgabe des erfindungsgemässen Futterzusatzmittels verglichen mit den bekannten Entwicklungen eher niedriger liegen kann. Besondere Bedeutung kommt der zusätzlichen oralen Verabreichung von Methionin bei Schafen zu, da schwefelhaltige Aminosäuren einen erheblichen Einfluss auf die Wollbildung haben. Für ein Schaf mit einem Körpergewicht von etwa 50 kg entspricht die Dosierung des erfindungsgemässen Futterzusatzmittels etwa einer Menge, die eine zusätzliche Gabe von etwa 5 g Methionin bedeutet.

Die Dosierung von erfindungsgemässen Futterzusatzmitteln, die andere Aminosäuren als Methionin enthalten, kann durch Tierversuche ermittelt werden, wie sie beispielsweise in den folgenden Beispielen beschrieben werden.

*Beispiel 1*

89,4 g (0,6 Mol) Methionin wurden bei Raumtemperatur in 1500 ml Wasser durch Zugabe von 200 ml 1 n Salzsäure in Lösung gebracht. Anschliessend wurden unter Rühren 86,2 g (0,3 Mol) Zinksulfat ($Zn\ SO_4 \times 7\ H_2O$) zu der Lösung gegeben, wobei sich das Salz rasch auflöste. Die erhaltene, klare Lösung wurde durch tropfenweise Zugabe von 45%-iger Kalilauge auf pH 7,5 gebracht, wobei intensiv gerührt wurde. Nach beendeter Zugabe wurde noch 10 Minuten gerührt, der ausgefallene Niederschalg abgetrennt, mit Wasser gewaschen (2 × 300 ml) und bei 110°C getrocknet.

Die Ausbeute an Zink-Methionin-Komplex im Verhältnis 1:2 (Bruttoformel: $C_{10}\ H_{20}\ N_2\ O_4\ S_2\ Zn$) betrug 104,1 g (= 95,9% der Theorie). Das Produkt wurde durch analytische Routineverfahren auf den Gehalt an Zink und Methionin untersucht. Es wurden folgende Werte ermittelt: 18,5% Zink (Theorie: 18,15%) und 78,5% Methionin (Theorie: 81,85%).

*Beispiel 2*

29,8 g (0,2 Mol) Methionin wurden in 500 ml Wasser unter Zusatz von 40 ml 2 n Salzsäure bei Raumtemperatur in Lösung gebracht. Anschliessend wurden 8,14 g (0,1 Mol) Zinkoxid zu der Lösung gegeben, die ebenfalls unter Zusatz von verdünnter Salzsäure vollständig in Lösung gebracht wurden. Durch tropfenweise Zugabe einer 45%igen Kaliumhydroxidlösung wurde der Ansatz dann auf einen pH-Wert von 7,5 eingestellt. Der ausgefallene Komplex wurde abgetrennt, mit 600 ml Wasser gewaschen und bei 110°C getrocknet. Die Ausbeute betrug 33,0 g (= 91,2% der Theorie) und die Analyse ergab 18,4% Zink und 77,6% Methionin.

Durch zusätzliche, übliche Reinigungsoperationen kann der in den Beispielen 1 und 2 erhaltene Zink-Methionin-Komplex noch gereinigt werden, wenngleich dies für die erfindungsgemässen Zwecke nicht erforderlich ist. Aufgrund der in den Beispielen 1 und 2 gewählten Volumina- und Mengenverhältnisse setzte die Ausfällung des Niederschlags bereits vor Erreichen eines pH-Wertes von 7,5 ein.

*Beispiel 3*

Der gemäss Beispiel 1 hergestellte Zink-Methionin-Komplex wurde in einem Fütterungsversuch mit Kühen getestet.

Eine bewährte Methode bei der Bestimmung des Aminosäurebedarfs bei Tieren ist eine steigende Gabe der betreffenden Aminosäure mit parallel gezogenen Blutproben. Solange der Bedarf an dieser Aminosäure nicht gedeckt ist, bleibt das Niveau im Serum konstant. Sobald der Bedarf gedeckt ist (und eventuell eine andere Aminosäure limitierend wirkt), steigt der Gehalt im Blutserum deutlich an. Es sei hierzu auf Degussa-Informationsdienst für die Mischfutterindustrie 1/1979, Seiten 1 bis 5 verwiesen, wo die skizzierte Methode ausführlich beschrieben und der beschriebene Sachverhalt auf Seite 2 graphisch dargestellt ist.

Zur vergleichenden Prüfung der Schutzwirkung und der Verfügbarkeit der geschützten Aminosäuren beim Wiederkäuer bietet es sich demnach an, eine über den Bedarf des Tieres liegende Menge zu geben, so dass der zu erwartende Serumgehalt stets auf der ansteigenden Kurve (siehe graphische Darstellung in der oben angegebenen Literaturstelle) liegen sollte. Auf diese Weise können verschiedene pansendurchtretende Futterzusatzmittel mit definiertem Methioningehalt geprüft und beispielsweise mit den durch Labmageninfusion erreichten Serumwerten verglichen werden. Diese Verfahrensweise hat den Vorteil, dass mit der das Blutserum erreichenden Methioninmenge gleichzeitig der Schutz im Pansen und die Verfügbarkeit in den folgenden Abschnitten des Verdauungstraktes erfasst werden.

Da die Passagezeit durch die Vormägen und auch die Geschwindigkeit der Resorption bei Anwendung verschiedener Methioninformen differieren können, wurden bei den durchgeführten Versuchen häufige kleine Gaben bei oraler Verabreichung gewählt, während Infusionen kontinuierlich erfolgten.

Die bei den durchgeführten Versuchen verwendeten Methionin-Formen und -Mengen sowie die jeweils gemessenen Blutserumwerte sind in der folgenden Tabelle wiedergegeben. Die Behandlungsperioden der einzelnen Kühe betrugen jeweils 1 Woche. Die oralen Gaben erfolgten in sechs gleichen Portionen/Tag gleichmässig verteilt zwischen 6.00 Uhr und 18.30 Uhr.

*Tabelle 1*

| | Methionin-Gehalt im Serum* µg/ml | |
|---|---|---|
| Methionin-Form | Kuh 1 | Kuh 2 |
| — ohne Methionin-Gabe | 2,7 | 3,1 |
| — 60 g (in 6 Dosen) oral verabreichter Methionin-Komplex (1:2)/Tag entsprechend rd. 47 g DL-Methionin | 3,7 | 3,7 |
| — 10 g infundiertes DL-Methionin in den Labmagen/Tag | 7,6 | 11,7 |

* Jeder Wert resultierte aus dem Mittel von drei Analysen von drei Blutentnahme-Zeitpunkten. Bei den Versuchstieren handelte es sich um laktierende Kühe.

Die in der obigen Tabelle wiedergegebenen Ergebnisse zeigen, dass die orale Verabreichung des erfindungsgemässen Zink-Methionin-Komplexes den Gehalt an Methionin im Blutserum deutlich erhöht, was bei Verwendung von wasserlöslichem Methionin nicht der Fall ist. Allerdings werden die Blutserumgehalte nach Infusion von Methionin bei weitem nicht erreicht.

*Beispiel 4*

Die in Beispiel 3 beschriebenen Versuche wurden wiederholt, wobei der Zinkkomplex mit einer Matrix

aus gesättigten Triglyceriden vermischt war. Die Herstellung dieser Mischung erfolgte nach dem oben beschriebenen Sprühkristallisationsverfahren, bei dem die aus dem Zinkkomplex und den Triglyceriden hergestellte heisse Schmelze unter Druck gegen einen kalten Luftstrom versprüht wurde. Der Gehalt an Zinkkomplex betrug berechnet als Methionin 30%. Zum Vergleich wurde normales Methionin in gleicher Weise mit der genannten Matrix vermischt. Auch hier betrug der Gehalt an Methionin 30%.

Die erhaltenen Versuchsergebnisse sind in der folgenden Tabelle 2 wiedergegeben. Die Serum-Proben wurden am letzten Tag der jeweiligen Behandlungsperiode um 14.00 Uhr gezogen, also knapp einwöchiger Behandlungsdauer.

*Tabelle 2*

| Methionin-Form (Gaben pro Tag) | Methionin-Gehalt im Serum* µg/ml | |
|---|---|---|
| | Kuh 1 | Kuh 2 |
| — 150 g normal geschütztes Methionin, (30%ig) = 45 g Methionin | 2,6 | 2,7 |
| — 7,5 g infundiertes DL-Methionin in den Labmagen | 4,0 | 9,0 |
| — ohne Methionin-Gabe | 1,8 | 2,0 |
| — 150 g erfindungsgemäss geschütztes Methionin (30%ig) = 45 g Methionin | 6,0 | 10,0 |
| — 15 g infundiertes DL-Methionin in den Labmagen | 8,0 | 12,0 |

* Jeder Wert resultierte aus dem Mittelwert von zwei Analysen. Bei den Versuchstieren handelte es sich wiederum um laktierende Kühe.

Die obigen Ergebnisse zeigen, dass der Zink-Methionin-Komplex, der allein die Blutserumwerte nur begrenzt erhöhte, in Verbindung mit der Matrix aus gesättigten Triglyceriden eine ausserordentliche Steigerung der effektiv resorbierten Methioninmenge ergab. Ferner machen die obigen Ergebnisse deutlich, dass die Verwendung der Schutzmatrix aus gesättigten Triglyceriden allein eine vergleichsweise geringe Wirkung hat.

Die verwendete Schutzmatrix besass folgende Fettsäurezusammensetzung (gesättigte Fettsäuren):

| | | |
|---|---|---|
| $C_{16}$ | : | 20,6% |
| $C_{18}$ | : | 53,8% |
| $C_{20}$ | : | 13,8% |
| $C_{22}$ | : | 7,8% |
| andere | : | 4,0% |
| | | 100% |

*Beispiel 5*

Der gemäss Beispiel 2 hergestellte Zink-Methionin-Komplex wurde wie in Beispiel 4 beschrieben mit einer Matrix aus Di- und Triglyceriden vermischt und nach dem Sprühkristallisationsverfahren verarbeitet. Das so erhaltene Produkt wurde in der in Beispiel 3 beschriebenen Weise in einem Fütterungsversuch mit Kühen getestet, wobei zum Vergleich 5 weitere Produkte getestet wurden, bei denen es sich entweder um im Handel erhältliche Produkte oder nach dem Stand der Technik hergestellte Produkte handelte, von denen belegt oder beschrieben ist, dass sie pansenfest sind. Alle Produkte wurden in einer Dosierung verabreicht, dass die Kühe umgerechnet 45 g Methionin/Tag (aufgeteilt auf 6 Tagesgaben zu je 7,5 g) erhielten.

Als Versuchstiere dienten 2 Kühe mit mittlerer-niedriger Milchleistung (durchschnittlich 18 l bzw. 33 l/Tag). Die Kühe wurden stets gleich behandelt. Die beiden Kühe reagierten auf die Methionin-Gaben unterschiedlich deutlich (Kuh 2 zeigte eine stärkere Reaktion). Diese individuellen Unterschiede sind bekannt. Zwischen den Produkttypen war hinsichtlich der Rangfolge der im Serum «ankommenden» Mengen an Methionin eine gute Vergleichbarkeit gegeben.

Die Einzelwerte der durchgeführten Analysen (gezogen jeweils um 11 und um 14 Uhr) wiesen recht grosse analytische Schwankungen auf. Deshalb wurden mindestens 4 Einzelanalysen je Produkt und Kuh durchgeführt. Wie in Beispiel 3 betrugen die Behandlungsperioden der einzelnen Kühe jeweils eine Woche.

Neben dem mit D bezeichneten erfindungsgemässen Produkt wurden folgende weitere Produkte zu Vergleichszwecken untersucht:

Produkt A: Hydroxymethyl-Methionin-Calciumsalz (Degussa-Mepron)

Produkt B: Methionin-Hydroxy-Analog-Calciumsalz

Produkt C: Methionin, das mit einer Matrix aus Di- und Triglyceriden sowie einem Emulgator umhüllt war.

Produkt E: Erfindungsgemäss geschütztes Methionin, das ebenfalls mit einer Matrix aus Di- und Triglyceriden sowie einem Emulgator (wie bei Produkt C) umhüllt war.

Produkt E: Methionin, das mit einer Matrix aus überwiegend gesättigten Fettsäuren umhüllt war.

Produkt F: Methionin, das mit einer Matrix aus ebenfalls überwiegend gesättigten Fettsäuren umhüllt war.

Die Produkte A und B sind im Handel erhältlich. Anstelle von Methionin enthält das Produkt A Methionin, bei dem sich an der Aminogruppe eine Hydroxymethylgruppe befindet. Beim Produkt B handelt es sich um eine dem Methionin entsprechende Verbindung, bei dem allerdings die Aminogruppe durch

eine Hydroxygruppe ersetzt ist. In beiden Fällen werden die eingesetzten Verbindungen vom Tier jedoch in vivo zu Methionin umgewandelt. Bei den Produkten E und F handelte es sich um zwei ähnliche Typen, die sich nur geringfügig in der Fettsäurezusammensetzung der Matrix unterschieden.

Die erhaltenen Versuchsergebnisse sind in der folgenden Tabelle 3 wiedergegeben.

*Tabelle 3*

| Periode | Eingesetztes Methioninpräparat | Methioningehalt im Serum µg/ml Kuh 1 | Kuh 2 |
|---|---|---|---|
| 1 | ohne Methionin-Gabe | 1,7 | 2,4 |
| 2 | Produkt A | 2,1 | 2,9 |
| 3 | ohne Methionin-Gabe | 2,3 | 2,2 |
| 4 | Produkt B | 2,4 | 4,5 |
| 5 | Produkt C | 3,3 | 5,2 |
| 6 | ohne Methionin-Gabe | 2,5 | 2,7 |
| 7 | erfindungsgemässes Produkt D | 5,2 | 10,0 |
| 8 | ohne Methionin-Gabe | 2,4 | 3,6 |
| 9 | Produkt E | 2,8 | 4,9 |
| 10 | ohne Methionin-Gabe | 2,0 | 2,6 |
| 11 | Produkt F | 2,9 | 4,8 |
| 12 | ohne Methionin-Gabe | 2,3 | 3,3 |

Das durchschnittliche Methionin-Niveau im Serum während der Perioden ohne Methionin-Gabe lag bei Kuh 1 deutlich unter dem Niveau der Kuh 2. Der höhere Wert in der Periode 8 ohne Methionin-Gabe bei Kuh 2 kann auf einer Nachwirkung des eingesetzten erfindungsgemäss geschützten Methionins beruhen, denn der erste Einzelwert aus dieser Periode war deutlich erhöht und betrug 5,6 µg/ml.

Die in der obigen Tabelle wiedergegebenen Ergebnisse zeigen deutlich, dass von den 6 Formen des untersuchten geschützten Methionins die erfindungsgemässe Form die bei weitem besten Ergebnisse erbrachte und damit den übrigen Produkten weit überlegen war. Wie im Beispiel 4 zeigen auch die vorliegenden Ergebnisse, dass die Verwendung einer Schutzmatrix allein eine vergleichsweise geringe Wirkung hat.

**Patentansprüche**

1. Teilchenförmiges Futterzusatzmittel für Wiederkäuer auf Basis von wasserunlöslichen Zinkkomplexen einer oder mehrerer essentieller Aminosäuren, dadurch gekennzeichnet, dass es aus einer Mischung der wasserunlöslichen Zinkkomplexe einer oder mehrerer essentieller Aminosäuren mit einer im wesentlichen pansenbeständigen Matrix besteht.

2. Futterzusatzmittel nach Anspruch 1, dadurch gekennzeichnet, dass die pansenbeständige Matrix eine die Zinkkomplexteilchen umhüllende Beschichtung ist.

3. Futterzusatzmittel nach den Ansprüchen 1 und 2, dadurch gekennzeichnet, dass es sich bei den essentiellen Aminosäuren in den wasserunlöslichen Zinkkomplexen um Methionin, Histidin, Phenylalanin und/oder Valin handelt.

4. Futterzusatzmittel nach den Ansprüchen 1 bis 3, dadurch gekennzeichnet, dass der wasserunlöslichen Zinkkomplex

$$[H_3C - S - CH_2 - CH_2 - \underset{\displaystyle NH_2}{\underset{|}{CH}} - COO]_2\,Zn$$

ist.

5. Verfahren zur Herstellung eines teilchenförmigen Futterzusatzmittels für Wiederkäuer, dadurch gekennzeichnet, dass man eine essentielle Aminosäure unter Zusatz von Säure in Wasser löst, anschliessend unter Rühren ein wasserlösliches Zinksalz in einer Menge zusetzt, dass das molare Verhältnis von Aminosäure zu Zinksalz 2:1 beträgt, die erhaltene, klare Lösung unter intensivem Rühren durch Zusatz von Alkalilauge auf eine pH-Wert von 7,5 bringt, das Rühren nach beendeter Zugabe für eine kurze Zeit fortsetzt, den ausgefallenen Niederschlag abtrennt, mit Wasser wäscht und trocknet und dann in an sich bekannter Weise mit einer an sich bekannten im wesentlichen pansenbeständigen Matrix vermischt und/oder die Niederschlagsteilchen mit dieser Matrix beschichtet.

6. Verfahren nach Anspruch 5, dadurch gekennzeichnet, dass man als Aminosäure Methionin verwendet und

$$[H_3C - S - CH_2 - CH_2 - \underset{\displaystyle NH_2}{\underset{|}{CH}} - COO]_2\,Zn$$

als Niederschlag gewinnt.

**Revendications**

1. Additif alimentaire sous forme de particules pour ruminants, à base de complexes de zinc insolubles dans l'eau d'un ou plusieurs acides aminés

essentiels, caractérisé en ce qu'il est constitué d'un mélange de complexe de zinc insolubles dans l'eau d'un ou plusieurs acides aminés essentiels, avec une matrice résistant pratiquement à la panse.

2. Additif alimentaire selon la revendication 1, caractérisé en ce que la matrice résistant à la panse est un revêtement enrobant les particules de complexe de zinc.

3. Additif alimentaire selon les revendications 1 et 2, caractérisé en ce que pour les acides aminés essentiels dans les complexes de zinc insolubles dans l'eau, il s'agit de méthionine, d'histidine, de phénylalanine et/ou de valine.

4. Additif alimentaire selon l'une quelconque des revendications 1 à 3, caractérisé en ce que le complexe de zinc insoluble dans l'eau est

$$[H_3C - S - CH_2 - CH_2 - \underset{\displaystyle NH_2}{\underset{|}{CH}} - COO]_2\ Zn$$

5. Procédé de préparation d'un additif alimentaire sous forme de particules pour ruminants, caractérisé en ce qu'on dissout dans l'eau un acide aminé essentiel en ajoutant un acide, puis en agitant on ajoute un sel de zinc soluble dans l'eau en une quantité telle que le rapport molaire de l'acide aminé au sel de zinc soit de 2:1, on ajuste le pH de la solution limpide ainsi obtenue à une valeur de 7,5 par addition de lessive alcaline en agitant vigoureusement, on poursuit l'agitation pendant encore un peu de temps après la fin de l'addition de produit alcalin, on sépare le précipité formé, on lave avec de l'eau et on sèche, puis, d'une manière connue en soi, on mélange avec une matrice résistant à la panse, connue en soi, et/ou on enrobe les particules de précipité avec cette matrice.

6. Procédé selon la revendication 5, caractérisé en ce qu'on utilise la méthionine en tant qu'acide aminé et qu'on obtient

$$[H_3C - S - CH_2 - CH_2 - \underset{\displaystyle NH_2}{\underset{|}{CH}} - COO]_2\ Zn$$

comme précipité.

**Claims**

1. Particulate feed additive for ruminants on the basis of water-insoluble zinc complexes of one or more essential amino acids, characterized in that it consists of a mixture of the water-insoluble zinc complexes of one or more essential amino acids with a substantially rumen-proof matrix.

2. Feed additive according to claim 1, characterized in that the rumen-proof matrix is a coating enveloping the zinc complex particles.

3. Feed additive according to claims 1 and 2, characterized in that the essential amino acids in the water-insoluble zinc complexes are methionine, histidine, phenylalanine and/or valine.

4. Feed additive according to claims 1 to 3, characterized in that the water-insoluble zinc complex is

$$[H_3C - S - CH_2 - CH_2 - \underset{\displaystyle NH_2}{\underset{|}{CH}} - COO]_2\ Zn$$

5. Process for producing a particulate feed additive for ruminants, characterized in that an essential amino acid is dissolved in water, accompanied by the addition of acid, subsequently and accompanied by stirring a water-soluble zinc salt is added in an amount that the molar ratio of amino acid to zinc salt is 2:1, the clear solution obtained is brought to a pH-value of 7.5 by adding alkali lye and accompanied by intensive stirring, the stirring is continued for a short time, the precipitate is separated, washed with water and dried and then is mixed in per se known manner with a per se known substantially rumen-proof matrix and/or the precipitate particles are coated with this matrix.

6. Process according to claim 5, characterized in that methionine is used as amino acid and

$$[H_3C - S - CH_2 - CH_2 - \underset{\displaystyle NH_2}{\underset{|}{CH}} - COO]_2\ Zn$$

is recovered as precipitate.